# EUROPEAN PATENT APPLICATION

(11) **EP 4 809 929 A1**
(43) Date of publication of application: **23.09.2026**
(21) Application number: 24891246.1
(22) Date of filing: 01.11.2024
(51) Int. Cl.: A61B 6/08, A61B 6/00

(54) **X-RAY IMAGING DEVICE**

(30) Priority: 17.11.2023 JP 2023196207
(71) Applicant: SHIMADZU CORPORATION, Kyoto-shi, Kyoto 604-8511 (JP)
(72) Inventor: FURUHASHI, Naoya, Kyoto-shi, Kyoto 604-8511 (JP); OKUMURA, Hiroshi, Kyoto-shi, Kyoto 604-8511 (JP)
(74) Representative: Müller-Boré & Partner Patentanwälte PartG mbB
(86) International application number: PCT/JP2024/038999
(87) International publication number: WO 2025/105206

(57) **Abstract**

This X-ray imaging apparatus (100) comprises: a holding unit (40) configured to hold an X-ray irradiation unit (10) movably in a horizontal direction; an optical imaging unit (30) provided in the holding unit (40); and a display unit (41) provided in the holding unit (40), and is configured to be capable of switching a first display state of an optical image (2) displayed on the display unit (41) to a second display state in which an orientation of a subject (1) is rotated so as to match an orientation of the subject (1) in an X-ray image.

## Description

### Technical Field

The present invention relates to an X-ray imaging apparatus.

### Background Art

Conventionally, an X-ray imaging apparatus including an optical imaging unit is known. Such an X-ray imaging apparatus is disclosed in, for example, Japanese Unexamined Patent Application Publication No. 2012-147978.

The X-ray imaging apparatus described in Japanese Unexamined Patent Application Publication No. 2012-147978 includes a camera (optical imaging unit) provided in an X-ray tube. This X-ray imaging apparatus automatically executes positioning of the X-ray tube and adjustment of an X-ray irradiation field based on an image including a subject captured by the camera.

### Citation List

### Patent Literature

Patent Literature 1: Japanese Unexamined Patent Application Publication No. 2012-147978

### Summary of Invention

### Technical Problem

Although not described in Japanese Unexamined Patent Application Publication No. 2012-147978, when performing X-ray imaging, an operator who performs the X-ray imaging, such as a radiologic technologist or a doctor (hereinafter sometimes referred to as an "operator"), adjusts the position of an X-ray irradiation unit and the body orientation of the subject with reference to an optical image including the subject captured by the camera (optical imaging unit) and displayed on a display unit. When performing X-ray imaging in a posture in which the subject is laid on a radiography table (decubitus position), the operator stands near the longitudinal side edge portion of the radiography table and adjusts the position of the X-ray irradiation unit and the body orientation of the subject while referring to the optical image displayed on the display unit. At this time, the longitudinal direction of the radiography table in the optical image is displayed along the left-right direction, similarly to when the operator visually observes the radiography table directly. However, in an X-ray image visually recognized during a diagnosis by a doctor, the longitudinal direction of the radiography table, which is generally a head-to-foot direction, is displayed along an up-down direction. Therefore, even if a radiologic technologist who provides an X-ray image suitable for diagnosis to a doctor, or a doctor who performs imaging and diagnosis, refers to the optical image displayed on the display unit after the adjustment of the position of the X-ray irradiation unit and the body orientation of the subject, it may be difficult to imagine the finished X-ray image to be captured. Therefore, it is desired that the operator can easily imagine the X-ray image to be captured based on the optical image displayed on the display unit.

The present invention has been made to solve the above-described problems, and one object of the present invention is to provide an X-ray imaging apparatus capable of allowing an operator to easily imagine an X-ray image to be captured based on an optical image displayed on a display unit.

### Solution to Problem

An X-ray imaging apparatus according to one aspect of the present invention includes: an X-ray irradiation unit including an X-ray tube; an X-ray detection unit configured to detect X-rays irradiated from the X-ray irradiation unit; a holding unit configured to hold the X-ray irradiation unit movably in a horizontal direction; an image generation unit configured to generate an X-ray image based on the X-rays detected by the X-ray detection unit; an optical imaging unit provided in the holding unit and configured to capture an optical image of a subject placed on a radiography table; and a display unit provided in the holding unit and configured to display the optical image captured by the optical imaging unit, wherein the X-ray imaging apparatus is configured to be capable of switching a first display state of the optical image displayed on the display unit to a second display state in which an orientation of the subject is rotated so as to match an orientation of the subject in the X-ray image generated by the image generation unit.

### Advantageous Effects of Invention

In the X-ray imaging apparatus according to the one aspect, as described above, the apparatus is configured to be capable of switching the first display state of the optical image displayed on the display unit provided in the holding unit to the second display state in which the orientation of the subject is rotated so as to match the orientation of the subject in the X-ray image generated by the image generation unit. As a result, after adjusting the position of the holding unit that holds the X-ray irradiation unit and the body orientation of the subject, and before X-ray imaging, the operator can visually recognize the optical image in which the displayed orientation of the subject is matched with the orientation of the subject in the X-ray image generated by the image generation unit. Therefore, the operator can easily imagine the X-ray image to be captured based on the optical image displayed on the display unit.

### Brief Description of Drawings

[FIG. 1] FIG. 1 is a schematic diagram illustrating an overall configuration of an X-ray imaging apparatus according to an embodiment.
[FIG. 2] FIG. 2 is a block diagram illustrating the overall configuration of the X-ray imaging apparatus according to the embodiment.
[FIG. 3] FIG. 3 is a schematic diagram illustrating a configuration of a holding unit according to the embodiment.
[FIG. 4] FIG. 4 is a schematic diagram of an operation display unit according to the embodiment.
[FIG. 5] FIG. 5 is a schematic diagram of a first display state of an optical image displayed on the operation display unit and a second display state of the optical image displayed on the operation display unit.
[FIG. 6] FIG. 6 is a schematic diagram for explaining a first example of switching control between the first display state and the second display state.
[FIG. 7] FIG. 7 is a schematic diagram for explaining a second example of the switching control between the first display state and the second display state.

### Description of Embodiments

Hereinafter, embodiments embodying the present invention will be described with reference to the drawings.

### (Configuration of X-ray Imaging Apparatus)

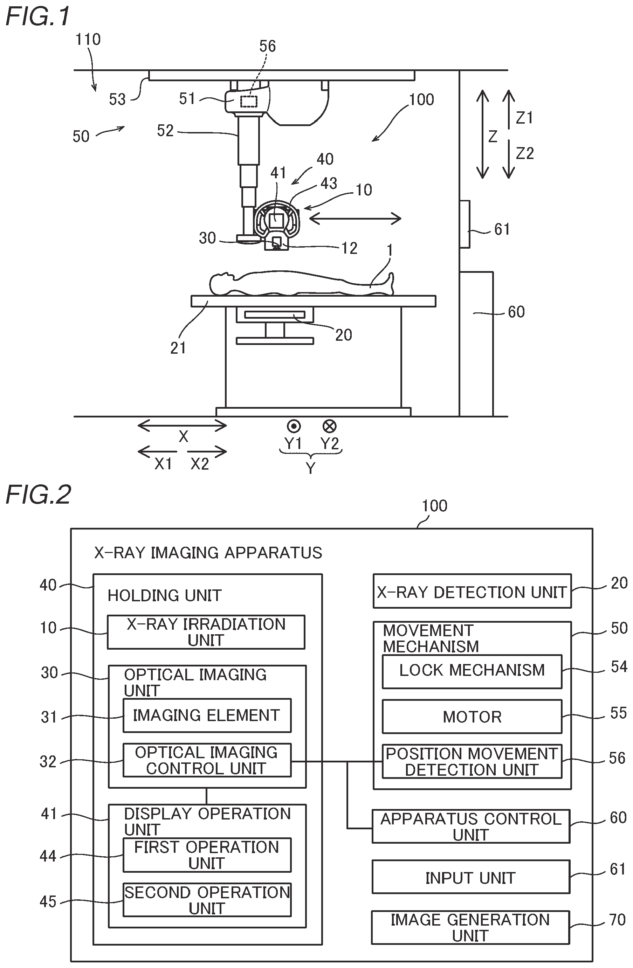

A configuration of an X-ray imaging apparatus 100 according to an embodiment of the present invention will be described with reference to FIGS. 1 to 4.

FIG. 1 shows an example of a ceiling-suspended X-ray imaging apparatus 100 installed in an imaging room 110. The X-ray imaging apparatus 100 includes an X-ray irradiation unit 10, an X-ray detection unit 20, an image generation unit 70, an optical imaging unit 30, a holding unit 40, a movement mechanism 50, an apparatus control unit 60, and an input unit 61. In the ceiling-suspended X-ray imaging apparatus 100, the holding unit 40 provided with the X-ray irradiation unit 10 and the optical imaging unit 30 is held so as to be suspended from a ceiling by the movement mechanism 50 arranged on the ceiling of the imaging room 110. The holding unit 40 is movably held in the imaging room 110 by the movement mechanism 50. Note that a vertical direction is a Z direction, a longitudinal direction of a radiography table 21 among horizontal directions is an X direction, and a transverse direction of the radiography table 21 among horizontal directions is a Y direction.

The X-ray imaging apparatus 100 is a medical X-ray imaging apparatus and is configured to perform X-ray imaging of a subject 1, which is an imaging target. The X-ray imaging apparatus 100 includes the radiography table 21 for performing imaging in a posture in which the subject 1 is laid (decubitus position).

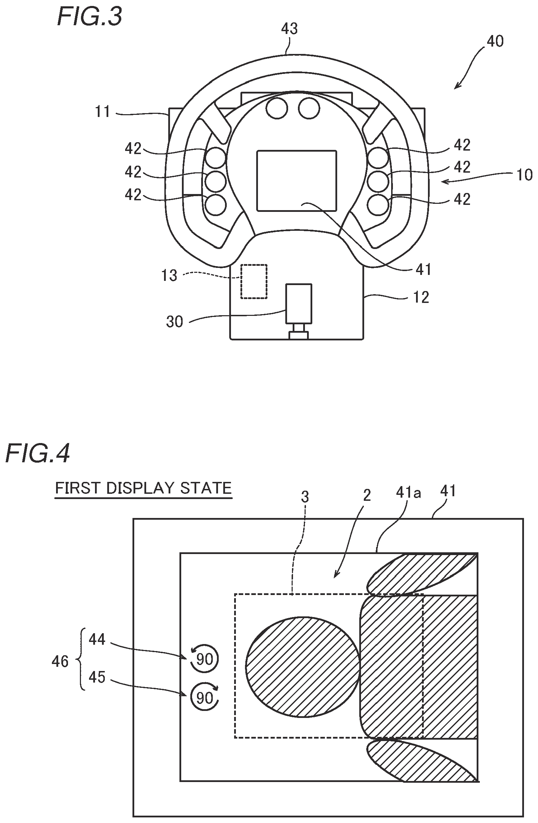

The X-ray irradiation unit 10 includes an X-ray tube 11 (see FIG. 3), a collimator 12, and an irradiation field lamp 13 (see FIG. 3). The X-ray tube 11 is configured to irradiate the subject 1 with X-rays. The X-ray tube 11 is configured to irradiate X-rays by applying a predetermined voltage. The collimator 12 is configured to adjust an irradiation field of the X-rays irradiated from the X-ray tube 11. The collimator 12 is provided in the vicinity of the X-ray tube 11 in an X-ray irradiation direction of the X-ray tube 11. The irradiation field lamp 13 is provided in the collimator 12. The irradiation field lamp 13 includes a visible light source. Visible light irradiated from the irradiation field lamp 13 allows the X-ray irradiation field to be confirmed without using X-rays.

The X-ray detection unit 20 is movably held on the radiography table 21. The X-ray detection unit 20 includes, for example, a flat panel detector (FPD). The X-ray detection unit 20 is configured to detect X-rays transmitted through the subject 1. In the decubitus X-ray imaging, the holding unit 40 is disposed at a position vertically facing the X-ray detection unit 20 of the radiography table 21, and the subject 1 lying on the radiography table 21 is imaged between the X-ray irradiation unit 10 and the X-ray detection unit 20 that vertically face each other.

The image generation unit 70 (see FIG. 2) is configured to generate an X-ray image based on the X-rays detected by the X-ray detection unit 20. The image generation unit 70 includes, for example, a CPU (Central Processing Unit), an FPGA (Field-Programmable Gate Array), and the like.

The optical imaging unit 30 is configured to capture an optical image 2 (see FIG. 4). As shown in FIG. 2, the optical imaging unit 30 includes an imaging element 31 and an optical imaging control unit 32. The optical imaging unit 30 is, for example, an optical camera. In the decubitus X-ray imaging, the holding unit 40 is disposed at a position vertically facing the X-ray detection unit 20 of the radiography table 21, and the subject 1 lying on the radiography table 21 is imaged by the optical imaging unit 30 between the X-ray irradiation unit 10 and the X-ray detection unit 20 that vertically face each other.

The imaging element 31 includes, for example, a CCD (Charge Coupled Device) image sensor or a CMOS (Complementary Metal Oxide Semiconductor) image sensor.

The optical imaging control unit 32 is a microcomputer including, for example, a CPU (Central Processing Unit) and a memory. The optical imaging control unit 32 is configured to receive a signal output from the imaging element 31 and generate the optical image 2 based on the input signal. As shown in FIG. 4, the generated optical image 2 is the optical image 2 in a first display state in which the head-to-foot direction of the subject 1 is displayed along a left-right direction of a display operation unit 41.

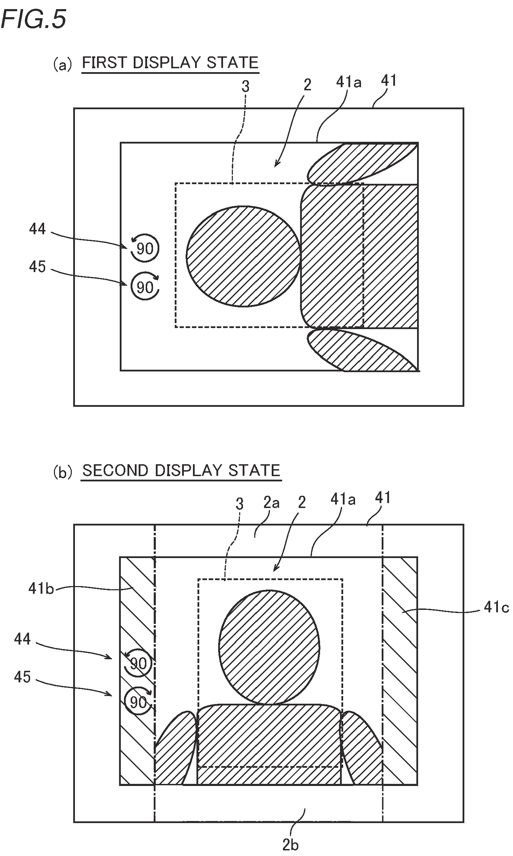

The optical imaging control unit 32 is configured to be capable of switching the first display state of the optical image 2 displayed on the display operation unit 41 to a second display state in which the orientation of the subject 1 is rotated so as to match the orientation of the subject 1 in the X-ray image generated by the image generation unit 70. In this embodiment, the optical imaging control unit 32 is configured to be capable of switching from the first display state of the optical image 2 shown in FIG. 5(a) in which the head-to-foot direction of the subject 1 is displayed along the left-right direction of the display operation unit 41, to the second display state shown in FIG. 5(b) in which the orientation of the subject 1 is rotated such that the head-to-foot direction of the subject 1 is represented along an up-down direction of the display operation unit 41. That is, the optical imaging control unit 32 is configured to control switching between the first display state and the second display state. The optical imaging control unit 32 is configured to output either the optical image 2 in the first display state or the optical image 2 in the second display state to the display operation unit 41. Then, the real-time optical image 2 output by the optical imaging control unit 32 is displayed on the display operation unit 41. The optical imaging control unit 32 is an example of a "control unit" in the claims.

Specifically, it is configured such that the optical image 2 is displayed on the display operation unit 41 in the first display state when adjusting a horizontal position of the holding unit 40, and the optical image 2 is displayed on the display operation unit 41 by switching from the first display state to the second display state after adjusting the horizontal position of the holding unit 40. The switching control between the first display state and the second display state by the optical imaging control unit 32 will be described later.

As shown in FIG. 1, the optical imaging unit 30 is mounted on the collimator 12. The optical imaging unit 30 is mounted facing an irradiation direction of X-rays from the X-ray irradiation unit 10. The optical imaging unit 30 can capture the optical image 2 (see FIG. 4) in directions of the subject 1 and the X-ray detection unit 20 from a side of the X-ray irradiation unit 10 when the X-ray irradiation unit 10 faces the directions of the subject 1 and the X-ray detection unit 20. The optical image 2 captured by the optical imaging unit 30 is an image captured from substantially the same direction as an imaging direction of the X-ray image. An imaging range of the optical imaging unit 30 is set to include a range of the X-ray irradiation field and to be larger than the range of the X-ray irradiation field.

As shown in FIG. 4, the optical imaging unit 30 can capture the optical image 2 including a visible light region 3 formed by visible light irradiated from the irradiation field lamp 13 (see FIG. 3) and a peripheral portion of the visible light region 3. A range of the visible light region 3 formed by the visible light irradiated from the irradiation field lamp 13 substantially coincides with the range of the X-ray irradiation field. The optical image 2 captured by the optical imaging unit 30 is displayed on the display operation unit 41. By using the visible light irradiated from the irradiation field lamp 13, the irradiation field of X-rays can be confirmed based on the optical image 2 without irradiating X-rays.

As shown in FIG. 1, the holding unit 40 holds the X-ray irradiation unit 10 movably. As shown in FIG. 4, the holding unit 40 includes the X-ray irradiation unit 10, the optical imaging unit 30, the display operation unit 41, a plurality of lock operation buttons 42, and a gripping unit 43. The holding unit 40 is configured to be movable in the horizontal direction and the vertical direction via the movement mechanism 50 by manual movement or control by the apparatus control unit 60.

The display operation unit 41 includes, for example, a touch panel type liquid crystal display. As shown in FIG. 4, the display operation unit 41 is configured to function as an image display unit including an image display area 41a in which the optical image 2 captured by the optical imaging unit 30, imaging information, and the like are displayed, and as an operation unit into which various operations by an operator are input. The display operation unit 41 is an example of a "display unit" in the claims. Note that, in FIG. 4, the optical image 2 in the first display state is displayed on the display operation unit 41.

The display operation unit 41 includes an operation unit 46 having a first operation unit 44 (see FIG. 4) and a second operation unit 45 (see FIG. 4). The first operation unit 44 is configured to receive an operation input for switching the first display state to the second display state in which the orientation of the subject 1 is rotated clockwise so as to match the orientation of the subject 1 in the X-ray image generated by the image generation unit 70. In this embodiment, the first operation unit 44 is configured to receive an operation input for switching from the first display state to the second display state in which the orientation of the subject 1 is rotated clockwise such that the head-to-foot direction of the subject 1 is represented along the up-down direction of the display operation unit 41.

The second operation unit 45 is configured to receive an operation input for switching the first display state to the second display state in which the orientation of the subject 1 is rotated counterclockwise so as to match the orientation of the subject 1 in the X-ray image generated by the image generation unit 70. In this embodiment, the second operation unit 45 is configured to receive an operation input for switching from the first display state to the second display state in which the orientation of the subject 1 is rotated counterclockwise such that the head-to-foot direction of the subject 1 is represented along the up-down direction of the display operation unit 41.

The plurality of lock operation buttons 42 are physical buttons pressed by an operator in order to control lock mechanisms 54, which will be described later, provided plurally in the movement mechanism 50. The lock mechanism 54 switches between a lock release state (a state permitting movement) and a lock state (a state prohibiting movement) of the holding unit 40 in each of a plurality of directions under control of the apparatus control unit 60. A plurality of lock operation buttons 42 are provided in the vicinity of the gripping unit 43 (at the gripping unit 43 or near the gripping unit 43) of the holding unit 40.

The gripping unit 43 is provided for an operator to grip when performing an operation of manually moving the holding unit 40. The gripping unit 43 transmits an operating force of the operator to the holding unit 40.

As shown in FIG. 1, the movement mechanism 50 is configured to movably hold the holding unit 40. The movement mechanism 50 includes a ceiling suspender 51 and a support pillar unit 52. The movement mechanism 50 is supported by rails 53 provided on the ceiling of the imaging room 110. The ceiling suspender 51 is configured to be movable in horizontal directions (X direction and Y direction) by the rails 53. The ceiling suspender 51 is configured to support the support pillar unit 52. The support pillar unit 52 is configured to support the holding unit 40. The support pillar unit 52 is configured to be vertically extendable and retractable (Z direction). The holding unit 40 is configured to be movable in the vertical direction by the support pillar unit 52.

Further, as shown in FIG. 2, the movement mechanism 50 includes the lock mechanisms 54 that restrict movement of the holding unit 40 in each direction (a plurality of directions including the X direction, the Y direction, and the Z direction), motors 55 that move the holding unit 40 in each direction (a plurality of directions including the X direction, the Y direction, and the Z direction), and a position movement detection unit 56.

A plurality of electromagnetic locks (electromagnetic brakes) are provided as the lock mechanisms 54. The lock mechanism 54 may be a hydraulic brake or a mechanical brake. Each electromagnetic lock is configured to releasably lock the movement of the holding unit 40 in each of the plurality of directions. The electromagnetic locks are individually provided for the plurality of directions including the X direction, the Y direction, and the Z direction. Each electromagnetic lock can individually switch between locking/unlocking in each of the plurality of directions including the X direction, the Y direction, and the Z direction. Accordingly, the lock mechanism 54 is configured to be switchable between a state permitting movement of the holding unit 40 in each of the plurality of directions (lock release state) and a state prohibiting movement of the holding unit 40 in each direction (lock state). Operations of the lock mechanisms 54 are controlled by the apparatus control unit 60.

The motors 55 are individually provided for a plurality of directions including the X direction, the Y direction, and the Z direction. The operation of each motor 55 is controlled by the apparatus control unit 60.

The position movement detection unit 56 includes a potentiometer. In addition, the position movement detection unit 56 is configured to detect movement of the holding unit 40 by electrically outputting a length of an extended or retracted wire due to the movement of the holding unit 40, and also to detect an absolute position of the holding unit 40.

The apparatus control unit 60 controls X-ray imaging by the X-ray irradiation unit 10 and the X-ray detection unit 20, and controls movement of the holding unit 40. The apparatus control unit 60 is configured to control switching between the lock release state and the lock state in the lock mechanisms 54 based on an input operation of the plurality of lock operation buttons 42. Furthermore, the apparatus control unit 60 is configured to control the movement of the holding unit 40. Specifically, the apparatus control unit 60 is configured to perform control for moving the holding unit 40 to a predetermined position and posture in auto-positioning control, and also to perform control for providing power assist to move the holding unit 40 when an operator manually moves the holding unit 40 in power assist control. The apparatus control unit 60 includes a CPU (Central Processing Unit) and a memory.

The input unit 61 has a function of receiving input operations related to X-ray imaging. The input operations include setting imaging conditions for X-ray imaging and issuing an instruction to start X-ray irradiation.

### (First Display State and Second Display State)

The first display state and the second display state of the optical image 2 will be described with reference to FIGS. 1 and 5.

The optical imaging unit 30 captures an image of the subject 1 lying on the radiography table 21. Here, as described above, when performing X-ray imaging in a posture in which the subject 1 is laid on the radiography table 21 (decubitus position), an operator stands near the longitudinal side edge portion of the radiography table 21 and adjusts the position of the X-ray irradiation unit 10 and the body orientation of the subject 1 while referring to the optical image 2 displayed on the display operation unit 41. At this time, since the subject 1 is placed on the radiography table 21 so that the head-to-foot direction of the subject 1 is along the longitudinal direction of the radiography table 21, the head-to-foot direction of the subject 1 is along the left-right direction in the visual field of the operator. Therefore, the head-to-foot direction of the subject 1 in the optical image 2 is captured along the left-right direction of the display operation unit 41 so as to coincide with the actual head-to-foot direction of the subject 1 viewed from the operator. That is, as shown in FIG. 5(a), the optical image 2 is captured such that the head-to-foot direction of the subject 1 is displayed along the left-right direction of the display operation unit 41. The first display state is a state in which the head-to-foot direction of the subject 1 is displayed along the left-right direction of the display operation unit 41 in the optical image 2 displayed on the display operation unit 41, and is a state in which the orientation of the subject 1 displayed on the display operation unit 41 does not match the orientation of the subject in the X-ray image generated by the image generation unit 70.

On the other hand, as shown in FIG. 5(b), the second display state is a state in which the head-to-foot direction of the subject 1 is displayed along the up-down direction of the display operation unit 41 in the optical image 2 displayed on the display operation unit 41. More specifically, the second display state is a state in which a direction from the head to the legs of the subject 1 is displayed along a direction from top to bottom of the display operation unit 41 in the optical image 2 displayed on the display operation unit 41. That is, the second display state is a state in which the up-down direction of the display operation unit 41 and the head-to-foot direction of the subject 1 substantially match each other, as shown at the time of diagnosis by a doctor, and is a state in which the orientation of the subject 1 displayed on the display operation unit 41 is matched with the orientation of the subject in the X-ray image generated by the image generation unit 70.

In this embodiment, the aspect ratio of the optical image 2 captured by the optical imaging unit 30 and the aspect ratio of the image display area 41a where the optical image 2 is displayed in the display operation unit 41 are both 4:3. Therefore, the optical image 2 displayed in the image display area 41a in the first display state is displayed in the image display area 41a without going out of the image display area 41a and being cut off. On the other hand, the optical image 2 displayed in the image display area 41a in the second display state is displayed in the image display area 41a with an upper end portion 2a and a lower end portion 2b extending outside the image display area 41a and being cut off. For convenience of explanation, in FIG. 5(b), the outer periphery of the optical image 2 in the second display state is indicated by an alternate long and short dash line.

However, the visible light region 3 formed by the visible light irradiated from the irradiation field lamp 13 in the center of the optical image 2 is located at a substantially central portion of the optical image 2. Therefore, in both the optical image 2 displayed in the image display area 41a in the first display state and the optical image 2 displayed in the image display area 41a in the second display state, the visible light region 3 is displayed in the image display area 41a without going out of the image display area 41a and being cut off. A left end portion 41b and a right end portion 41c of the image display area 41a in which the optical image 2 of the second display state is not displayed are painted, for example, in black (indicated by hatching for convenience of explanation).

When switching between the display on the display operation unit 41 in the first display state and the display on the display operation unit 41 in the second display state, the optical image 2 is displayed on the display operation unit 41 without being enlarged or reduced. As a result, it is possible to suppress an visual discomfort caused by a change in size of the subject 1 displayed on the display operation unit 41 during switching between the first display state and the second display state. The aspect ratio of the optical image 2 captured by the optical imaging unit 30 and the aspect ratio of the display operation unit 41 are not particularly limited.

### (Timing of Switching Between First Display State and Second Display State)

With reference to FIG. 5(a) and FIG. 5(b), timings of switching between display of the optical image 2 in the first display state on the display operation unit 41 and display of the optical image 2 in the second display state on the display operation unit 41 will be described. In the following description, "adjustment" refers to adjustment of the horizontal position of the holding unit 40.

The optical imaging control unit 32 is configured to control the display operation unit 41 to display the optical image 2 by switching from the first display state during adjustment to the second display state after adjustment, based on at least one of an input operation before or after the adjustment and a first moving operation of the holding unit 40 after the adjustment.

Here, the input operation includes an input operation to the display operation unit 41. Specifically, the input operation is an input operation to the first operation unit 44 or the second operation unit 45 of the display operation unit 41. The optical imaging control unit 32 displays the optical image 2 on the display operation unit 41 by switching from the first display state to the second display state based on the input operation to the first operation unit 44 or the second operation unit 45.

Also, the first moving operation includes an operation of stopping movement of the holding unit 40 in the horizontal direction. Specifically, the operation of stopping the movement of the holding unit 40 in the horizontal direction as the first moving operation means that manual movement of the holding unit 40 in the horizontal direction has been stopped, or that movement of the holding unit 40 in the horizontal direction via the movement mechanism 50 by control of the apparatus control unit 60 has been stopped. Based on the detection result of the position movement detection unit 56 provided in the movement mechanism 50, the optical imaging control unit 32 switches the optical image 2 from the first display state to the second display state and displays it on the display operation unit 41 when horizontal movement of the holding unit 40 is stopped.

The optical imaging control unit 32 is configured to display the optical image 2 on the display operation unit 41 by switching from the second display state based on an input operation before adjustment to the first display state during adjustment, based on a second moving operation of the holding unit 40 during adjustment.

Furthermore, the optical imaging control unit 32 switches from the second display state, which was switched based on an input operation before adjustment, to the first display state during adjustment to display the optical image 2 on the display operation unit 41 based on the second moving operation of the holding unit 40 during adjustment. Additionally, the optical imaging control unit 32 is configured to switch from the first display state during adjustment to the second display state after adjustment based on the first moving operation after adjustment, and display the optical image 2 on the display operation unit 41.

Here, the second moving operation includes a moving operation of the holding unit 40 in the horizontal direction. Specifically, the operation of moving the holding unit 40 in the horizontal direction as the second moving operation refers to an operation of manual movement of the holding unit 40 in the horizontal direction, or an operation of movement of the holding unit 40 in the horizontal direction via the movement mechanism 50 under the control of the apparatus control unit 60. The optical imaging control unit 32 switches from the second display state to the first display state and displays the optical image 2 on the display operation unit 41 when the horizontal moving operation of the holding unit 40 is being executed based on the detection result of the position movement detection unit 56 provided in the movement mechanism 50.

### (Switching Control Between First Display State and Second Display State)

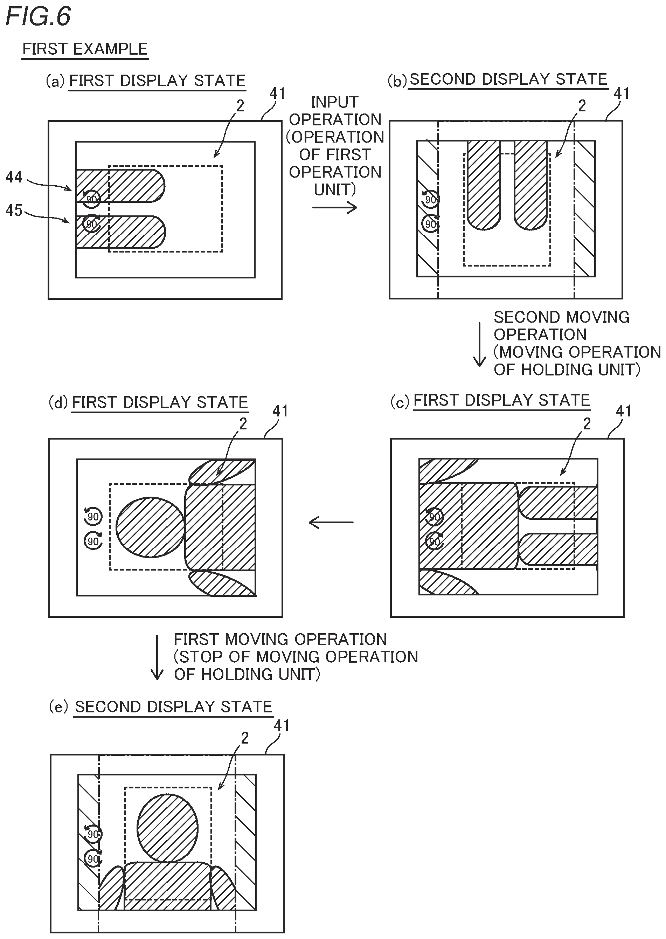

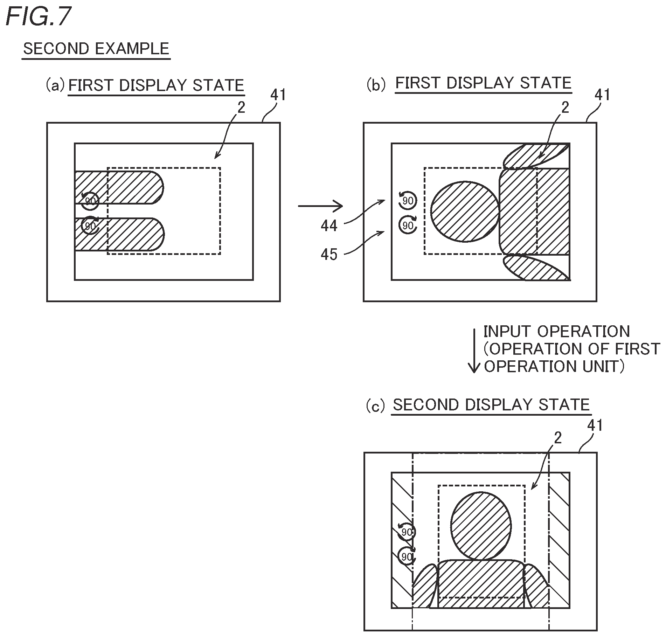

An example of switching control between the first display state and the second display state by the optical imaging control unit 32 will be described with reference to FIGS. 6 and 7.

In the following example, when performing X-ray imaging in a posture in which the subject 1 is laid on the radiography table 21 (decubitus position), a case will be described where the position of the holding unit 40 is adjusted by horizontally moving the holding unit 40 provided with the X-ray irradiation unit 10 to a position corresponding to the imaging region of the subject 1, and the state is switched from the first display state to the second display state after adjusting the horizontal position of the holding unit 40. Here, as an example, the imaging region of the subject 1 is the head of the subject 1. Also, as an example, the holding unit 40 before adjustment is positioned substantially directly above the lower limbs of the subject 1. Furthermore, in the visual field of the operator, the subject 1 is placed on the radiography table 21 such that the head-to-foot direction of the subject 1 is along the left-right direction and the head of the subject faces the left side.

### <First Example>

A first example of the switching control will be described with reference to FIGS. 6(a) to 6(e). FIG. 6(a) shows the optical image 2 displayed on the display operation unit 41 before adjustment. The optical imaging control unit 32 generates the optical image 2 based on the signal output by the imaging element 31. Since the holding unit 40 provided with the optical imaging unit 30 is positioned substantially directly above the lower limbs of the subject 1, the optical imaging control unit 32 generates the optical image 2 of the lower limbs of the subject 1 in the first display state, and outputs the optical image 2 of the lower limbs of the subject 1 in the first display state to the display operation unit 41. As a result, the optical image 2 of the lower limbs of the subject 1 in the first display state is displayed on the display operation unit 41.

Then, the operator operates the first operation unit 44. As a result, as shown in FIG. 6(b), the optical image 2 displayed on the display operation unit 41 is switched from the first display state to the second display state in which the orientation of the subject 1 is rotated so as to match the orientation of the subject 1 in the X-ray image generated by the image generation unit 70. Specifically, the optical image 2 displayed on the display operation unit 41 is switched from the first display state to the second display state in which the orientation of the subject 1 is rotated clockwise such that the head-to-foot direction of the subject 1 is represented along the up-down direction of the display operation unit 41. That is, the optical imaging control unit 32 switches from the first display state to the second display state based on the input operation to the first operation unit 44 before adjustment, and displays the optical image 2 on the display operation unit 41.

Then, the operator moves the holding unit 40 in a left direction where the head of the subject 1 is located. The movement of the holding unit 40 may be either an operation of manual horizontal movement of the holding unit 40, or an operation of horizontal movement of the holding unit 40 via the movement mechanism 50 under the control of the apparatus control unit 60. As a result, as shown in FIG. 6(c), the optical image 2 displayed on the display operation unit 41 is switched from the second display state to the first display state. That is, the optical imaging control unit 32 switches from the second display state switched based on the input operation before adjustment to the first display state during adjustment, based on the second moving operation serving as the horizontal moving operation of the holding unit 40 as a detection result of the position movement detection unit 56 provided in the movement mechanism 50, and displays the optical image 2 on the display operation unit 41.

Then, the operator performs adjustment by moving the holding unit 40 to a position corresponding to the head of the subject 1, and stops the movement of the holding unit 40 after the adjustment. FIG. 6(d) is the optical image 2 displayed on the display operation unit 41 immediately before the movement of the holding unit 40 is stopped after the adjustment.

Then, when the movement of the holding unit 40 is stopped after the adjustment, the optical image 2 displayed on the display operation unit 41 is switched from the first display state to the second display state, as shown in FIG. 6(e). That is, the optical imaging control unit 32 switches the state from the first display state during adjustment to the second display state after adjustment based on the first moving operation as a stop of the horizontal moving operation of the holding unit 40 serving as the detection result of the position movement detection unit 56 provided in the movement mechanism 50, and displays the optical image 2 on the display operation unit 41.

### <Second Example>

A second example of the switching control will be described with reference to FIGS. 7(a) to 7(c). FIG. 7(a) shows the optical image 2 displayed on the display operation unit 41 before adjustment. Similarly to the first example, the optical image 2 of the lower limbs of the subject 1 in the first display state is displayed on the display operation unit 41.

Then, the operator moves the holding unit 40 in the left direction where the head of the subject 1 is located. The movement of the holding unit 40 may be either an operation of manual horizontal movement of the holding unit 40, or an operation of horizontal movement of the holding unit 40 via the movement mechanism 50 under the control of the apparatus control unit 60. The optical image 2 of the subject 1 in the first display state is displayed on the display operation unit 41. Then, the operator performs adjustment by moving the holding unit 40 to a position corresponding to the head of the subject 1, and stops the movement of the holding unit 40 after adjustment, as shown in FIG. 7(b).

Then, the operator operates the first operation unit 44. As a result, as shown in FIG. 7(c), the optical image 2 displayed on the display operation unit 41 is switched from the first display state to the second display state in which the orientation of the subject 1 is rotated so as to match the orientation of the subject 1 in the X-ray image generated by the image generation unit 70. Specifically, the optical image 2 displayed on the display operation unit 41 is switched from the first display state to the second display state in which the orientation of the subject 1 is rotated clockwise such that the head-to-foot direction of the subject 1 is represented along the up-down direction of the display operation unit 41. That is, based on an input operation to the first operation unit 44 after adjustment, the optical imaging control unit 32 controls to switch from the first display state during adjustment to the second display state after adjustment and displays the optical image 2 on the display operation unit 41.

In either of the first example and the second example, in a case where the holding unit 40 is horizontally moved for readjustment after the optical image 2 is displayed on the display operation unit 41 by switching to the second display state after adjustment, as shown in FIG. 6(c), the optical imaging control unit 32 switches from the second display state to the first display state based on the second moving operation as the horizontal moving operation of the holding unit 40 as a detection result of the position movement detection unit 56 provided in the movement mechanism 50, and displays the optical image 2 on the display operation unit 41.

Then, when the movement of the holding unit 40 is stopped after readjustment, as shown in FIG. 6(e), the optical imaging control unit 32 switches from the first display state to the second display state based on the first moving operation as the stop of the horizontal moving operation of the holding unit 40 as a detection result of the position movement detection unit 56 provided in the movement mechanism 50, and displays the optical image 2 on the display operation unit 41.

Furthermore, in both the first example and the second example, after displaying the optical image 2 on the display operation unit 41 by switching to the second display state after adjustment, when the operator desires to display the optical image 2 on the display operation unit 41 by switching from the second display state to the first display state so as to match the actual head-to-foot direction of the subject 1 as seen by the operator, the operator operates the second operation unit 45. Thereby, it is possible to switch from the second display state to the first display state in which the orientation of the subject 1 is rotated counterclockwise so that the head-to-foot direction of the subject 1 is represented along the left-right direction of the display operation unit 41.

When the subject 1 is placed on the radiography table 21 such that the head-to-foot direction of the subject 1 is along the left-right direction and the head of the subject faces the right side in the operator's visual field, the operator operates the second operation unit 45 as an input operation before adjustment or after adjustment.

### (Effects of Present Embodiment)

In this embodiment, the following effects can be obtained.

In this embodiment, as described above, the apparatus is configured to be capable of switching the first display state of the optical image 2 displayed on the display operation unit 41 provided in the holding unit 40 to the second display state in which the orientation of the subject 1 is rotated so as to match the orientation of the subject in the X-ray image generated by the image generation unit 70. As a result, after adjusting the position of the holding unit 40 that holds the X-ray irradiation unit 10 and the body orientation of the subject 1, and before X-ray imaging, the operator can visually recognize the optical image 2 in which the displayed orientation of the subject 1 is matched with the orientation of the subject in the X-ray image generated by the image generation unit 70. Therefore, the operator can easily imagine the X-ray image to be captured based on the optical image 2 displayed on the display operation unit 41.

Further, in the above embodiment, the following additional effects are obtained by configuring as follows.

That is, in the present embodiment, as described above, when adjusting the horizontal position of the holding unit 40, the optical image 2 is displayed on the display operation unit 41 in the first display state, and after the adjustment, the optical image 2 is displayed on the display operation unit 41 by switching from the first display state to the second display state. As a result, when adjusting the horizontal position of the holding unit 40, the optical image 2 is displayed in the first display state such that the orientation of an imaging region of the subject 1 in the optical image 2 coincides with the orientation of an actual imaging region of the subject 1 as viewed from the operator. Further, after adjusting the horizontal position of the holding unit 40, the optical image 2 is displayed in the second display state in which the orientation of the displayed imaging region of the subject 1 matches the orientation of an imaging region of the subject 1 in an X-ray image shown at the time of diagnosis by a doctor. Therefore, when adjusting the horizontal position of the holding unit 40, the operator can easily move the holding unit 40 based on the optical image 2 displayed on the display operation unit 41, and after adjusting the position of the holding unit 40 and the body orientation of the subject 1, the operator can easily imagine the X-ray image to be captured based on the optical image 2 displayed on the display operation unit 41.

Further, in the present embodiment, as described above, the optical imaging control unit 32 that performs switching control between the first display state and the second display state is further provided, and the optical imaging control unit 32 is configured to perform control to display the optical image 2 on the display operation unit 41 by switching from the first display state during adjustment to the second display state after adjustment, based on at least one of an input operation before or after the adjustment and the first moving operation of the holding unit 40 after the adjustment. As a result, based on the input operation or the first moving operation, the operator can easily switch from the first display state during adjustment to the second display state after adjustment.

Further, in the present embodiment, as described above, the input operation includes an input operation to the operation unit 46 provided in the X-ray imaging apparatus 100, and the first moving operation includes an operation of stopping movement of the holding unit 40 in the horizontal direction. As a result, the input operation to the operation unit 46 enables the operator to more easily switch from the first display state during adjustment to the second display state after adjustment, and the horizontal movement stopping operation of the holding unit 40 can save the operator the trouble of performing a separate operation to switch from the first display state to the second display state.

In the present embodiment, as described above, the optical imaging control unit 32 is configured to display the optical image 2 on the display operation unit 41 by switching from the second display state to the first display state during adjustment, based on the second moving operation of the holding unit 40 during adjustment, starting from the second display state based on the input operation prior to the adjustment. As a result, since switching from the second display state to the first display state is performed based on the second moving operation of the holding unit 40 during adjustment, it is possible to save the operator the trouble of separately performing an operation of switching from the second display state to the first display state.

Further, in the present embodiment, as described above, the optical imaging control unit 32 displays the optical image 2 on the display operation unit 41 by switching from the second display state, which is switched based on the input operation before adjustment, to the first display state during adjustment, based on the second moving operation of the holding unit 40 during adjustment, and also displays the optical image 2 on the display operation unit 41 by switching from the first display state during adjustment to the second display state after adjustment, based on the first moving operation after adjustment. Because of this, switching from the second display state to the first display state is performed based on the second moving operation of the holding unit 40 during adjustment, and switching from the first display state to the second display state is performed based on the first moving operation after adjustment, which can save the operator the trouble of performing separate operations to switch between the second display state and the first display state.

Furthermore, in the present embodiment, as described above, the second moving operation includes a moving operation of the holding unit 40 in the horizontal direction. As a result, by performing the horizontal moving operation of the holding unit 40, it is possible to easily save the operator the trouble of performing a separate operation for switching from the second display state during adjustment to the first display state.

Further, in the present embodiment, as described above, an operation unit 46 that accepts an input operation is further provided, and the operation unit 46 includes a first operation unit 44 that switches the first display state to the second display state in which the orientation of the subject 1 is rotated clockwise so as to match the orientation of the subject 1 in the X-ray image generated by the image generation unit 70, and a second operation unit 45 that switches the first display state to the second display state in which the orientation of the subject 1 is rotated counterclockwise so as to match the orientation of the subject 1 in the X-ray image generated by the image generation unit 70. As a result, for example, regardless of whether the head of the subject faces rightwards or leftwards in the longitudinal direction of the radiography table in the visual field of the operator, the optical image 2 can be easily displayed on the display operation unit 41 in the second display state in which the orientation of the subject displayed on the display operation unit 41 matches the orientation of the subject in the X-ray image generated by the image generation unit.

### [Modifications]

It should be considered that the embodiments disclosed herein are illustrative and not restrictive in all aspects. The scope of the present invention is shown by the claims, not by the description of the above embodiments, and includes all modifications (modifications) within the meaning and scope equivalent to the claims.

For example, in the above embodiment, an example was shown in which the optical imaging control unit performs switching control between the first display state and the second display state and is configured to output either the optical image in the first display state or the optical image in the second display state to the display operation unit, but the present invention is not limited thereto. For example, the X-ray imaging apparatus may further include an optical imaging unit rotation mechanism capable of rotating the optical imaging unit clockwise and counterclockwise so as to be capable of capturing the optical image in the first display state and the optical image in the second display state. In this case, the optical image control unit may be configured to perform rotation control of the optical imaging unit by the optical imaging unit rotation mechanism to switch between capturing the optical image in the first display state and capturing the optical image in the second display state, or the apparatus control unit may be configured to perform rotation control of the optical imaging unit by the optical imaging unit rotation mechanism.

Further, instead of the optical imaging unit rotation mechanism, the optical imaging unit may further include an imaging element rotation unit capable of rotating the imaging element of the optical imaging unit clockwise and counterclockwise so as to be capable of capturing the optical image in the first display state and the optical image in the second display state. In this case, the optical image control unit may be configured to perform rotation control of the imaging element by the imaging element rotation unit for switching between capturing the optical image in the first display state and capturing the optical image in the second display state.

Further, in the above embodiment, an example was shown in which the optical imaging control unit including a microcomputer performs switching control between the first display state and the second display state, and is configured to output either the optical image of the first display state or the optical image of the second display state to the display operation unit, but the present invention is not limited to this. For example, an apparatus control unit that controls X-ray imaging and controls movement of the holding unit may be configured to acquire the optical image of the first display state generated by the optical image control unit, perform switching control between the first display state and the second display state for the acquired optical image, and display either the optical image of the first display state or the optical image of the second display state on the display operation unit.

Further, in the above embodiment, the first moving operation is stopping horizontal movement of the holding unit manually, or stopping horizontal movement of the holding unit via the moving mechanism controlled by the apparatus control unit, and the optical imaging control unit is configured to switch from the first display state to the second display state and display the optical image on the display operation unit when the horizontal movement of the holding unit is stopped based on the detection result of the position movement detection unit provided in the movement mechanism; the second moving operation is manual horizontal movement of the holding unit, or horizontal movement of the holding unit via the movement mechanism controlled by the apparatus control unit, and the optical imaging control unit is configured to switch from the second display state to the first display state and display the optical image on the display operation unit when the horizontal movement of the holding unit is executed based on the detection result of the position movement detection unit provided in the movement mechanism. However, the present invention is not limited to this.

For example, the first moving operation may be an operation for restricting horizontal movement of the holding unit instead of an operation for stopping horizontal movement of the holding unit. Specifically, the operation for restricting horizontal movement of the holding unit serving as the first moving operation may be an operation based on a lock operation button that switches to a lock state (a state prohibiting movement) of the holding unit for all of a plurality of directions including the X direction, the Y direction, and the Z direction by electromagnetic locks. The optical imaging control unit may be configured to display the optical image on the display unit by switching from the first display state to the second display state based on control of the lock state of the lock mechanism by the apparatus control unit.

In this case, the second moving operation may be an operation for releasing restriction of horizontal movement of the holding unit instead of horizontal movement of the holding unit. Specifically, the operation for releasing the horizontal movement restriction of the holding unit serving as the second moving operation may be an operation based on a lock operation button that switches to a lock release state (a state permitting movement) of the holding unit for all of a plurality of directions including the X direction, the Y direction, and the Z direction by electromagnetic locks. The optical imaging control unit may be configured to display the optical image on the display unit by switching from the second display state to the first display state based on control of the lock release state of the lock mechanism by the apparatus control unit. This also allows an operator to more easily switch between the first display state and the second display state.

Further, in the above embodiment, an example was shown in which the optical image control unit switches from the first display state during adjustment to the second display state after adjustment based on an input operation to the first operation unit or the second operation unit before or after adjustment, and displays the optical image on the display unit, but the present invention is not limited to this. For example, without input operations to the first operation unit and the second operation unit before or after adjustment, the optical imaging control unit may be configured to switch from the first display state to the second display state and display the optical image on the display operation unit when horizontal movement of the holding unit is stopped based on a detection result of the position movement detection unit provided in the movement mechanism, and switch from the second display state to the first display state and display the optical image on the display operation unit when a horizontal moving operation of the holding unit is executed.

Furthermore, in the above embodiment, an example was shown in which the X-ray imaging apparatus performs imaging in a posture where the subject is laid down (decubitus position), but the present invention is not limited to this. For example, the X-ray imaging apparatus may be configured to perform imaging in a state where a subject is seated on a chair or a wheelchair (sitting position) while the subject's arms are placed on a radiography table.

### [Aspects]

It will be understood by those skilled in the art that the exemplary embodiments described above are specific examples of the following aspects.

### (Item 1)

An X-ray imaging apparatus comprising: an X-ray irradiation unit including an X-ray tube; an X-ray detection unit configured to detect X-rays irradiated from the X-ray irradiation unit; a holding unit configured to hold the X-ray irradiation unit movably in a horizontal direction; an image generation unit configured to generate an X-ray image based on the X-rays detected by the X-ray detection unit; an optical imaging unit provided in the holding unit and configured to capture an optical image of a subject placed on a radiography table; and a display unit provided in the holding unit and configured to display the optical image captured by the optical imaging unit, wherein the X-ray imaging apparatus is configured to be capable of switching a first display state of the optical image displayed on the display unit to a second display state in which an orientation of the subject is rotated so as to match an orientation of the subject in the X-ray image generated by the image generation unit.

### (Item 2)

The X-ray imaging apparatus according to item 1, configured such that when adjusting a horizontal position of the holding unit, the optical image is displayed on the display unit in the first display state, and after the adjustment, the optical image is displayed on the display unit by switching from the first display state to the second display state.

### (Item 3)

The X-ray imaging apparatus according to item 2, further comprising a control unit configured to perform switching control between the first display state and the second display state, wherein the control unit is configured to perform control to display the optical image on the display unit by switching from the first display state during the adjustment to the second display state after the adjustment, based on at least one of an input operation before the adjustment or after the adjustment and a first moving operation of the holding unit after the adjustment.

### (Item 4)

The X-ray imaging apparatus according to item 3, wherein the input operation includes an input operation to an operation unit provided in the X-ray imaging apparatus, and the first moving operation includes at least one of an operation of stopping movement of the holding unit in the horizontal direction and an operation for restricting movement of the holding unit in the horizontal direction.

### (Item 5)

The X-ray imaging apparatus according to item 3 or 4, wherein the control unit is configured to display the optical image on the display unit by switching from the second display state to the first display state during the adjustment, based on a second moving operation of the holding unit during the adjustment, starting from the second display state based on the input operation prior to the adjustment.

### (Item 6)

The X-ray imaging apparatus according to any one of items 3 to 5, wherein the control unit is configured to display the optical image on the display unit by switching from the second display state to the first display state during the adjustment, based on a second moving operation of the holding unit during the adjustment, starting from the second display state switched based on the input operation prior to the adjustment, and is configured to display the optical image on the display unit by switching from the first display state during the adjustment to the second display state after the adjustment based on the first moving operation after the adjustment.

### (Item 7)

The X-ray imaging apparatus according to item 5 or 6, wherein the second moving operation includes a moving operation of the holding unit in the horizontal direction and an operation for releasing movement restriction of the holding unit in the horizontal direction.

### (Item 8)

The X-ray imaging apparatus according to any one of items 1 to 7, further comprising an operation unit configured to receive an input operation, wherein the operation unit includes a first operation unit configured to switch the first display state to the second display state in which the orientation of the subject is rotated clockwise so as to match the orientation of the subject in the X-ray image generated by the image generation unit, and a second operation unit configured to switch the first display state to the second display state in which the orientation of the subject is rotated counterclockwise so as to match the orientation of the subject in the X-ray image generated by the image generation unit.

### Reference Signs List

1 Subject
2 Optical image
10 X-ray irradiation unit
11 X-ray tube
20 X-ray detection unit
21 Radiography table
30 Optical imaging unit
32 Optical imaging control unit (control unit)
40 Holding unit
41 Display operation unit (display unit)
44 First operation unit
45 Second operation unit
46 Operation unit
70 Image generation unit
100 X-ray imaging apparatus

## Claims

1. An X-ray imaging apparatus comprising: an X-ray irradiation unit including an X-ray tube; an X-ray detection unit configured to detect X-rays irradiated from the X-ray irradiation unit; a holding unit configured to hold the X-ray irradiation unit movably in a horizontal direction; an image generation unit configured to generate an X-ray image based on the X-rays detected by the X-ray detection unit; an optical imaging unit provided in the holding unit and configured to capture an optical image of a subject placed on a radiography table; and a display unit provided in the holding unit and configured to display the optical image captured by the optical imaging unit, wherein the X-ray imaging apparatus is configured to be capable of switching a first display state of the optical image displayed on the display unit to a second display state in which an orientation of the subject is rotated so as to match an orientation of the subject in the X-ray image generated by the image generation unit.

2. The X-ray imaging apparatus according to claim 1, configured such that when adjusting a horizontal position of the holding unit, the optical image is displayed on the display unit in the first display state, and after the adjustment, the optical image is displayed on the display unit by switching from the first display state to the second display state.

3. The X-ray imaging apparatus according to claim 2, further comprising a control unit configured to perform switching control between the first display state and the second display state, wherein the control unit is configured to perform control to display the optical image on the display unit by switching from the first display state during the adjustment to the second display state after the adjustment, based on at least one of an input operation before the adjustment or after the adjustment and a first moving operation of the holding unit after the adjustment.

4. The X-ray imaging apparatus according to claim 3, wherein the input operation includes an input operation to an operation unit provided in the X-ray imaging apparatus, and the first moving operation includes at least one of an operation of stopping movement of the holding unit in the horizontal direction and an operation for restricting movement of the holding unit in the horizontal direction.

5. The X-ray imaging apparatus according to claim 3, wherein the control unit is configured to display the optical image on the display unit by switching from the second display state to the first display state during the adjustment, based on a second moving operation of the holding unit during the adjustment, starting from the second display state based on the input operation prior to the adjustment.

6. The X-ray imaging apparatus according to claim 3, wherein the control unit is configured to display the optical image on the display unit by switching from the second display state to the first display state during the adjustment, based on a second moving operation of the holding unit during the adjustment, starting from the second display state switched based on the input operation prior to the adjustment, and is configured to display the optical image on the display unit by switching from the first display state during the adjustment to the second display state after the adjustment based on the first moving operation after the adjustment.

7. The X-ray imaging apparatus according to claim 5, wherein the second moving operation includes a moving operation of the holding unit in the horizontal direction and an operation for releasing movement restriction of the holding unit in the horizontal direction.

8. The X-ray imaging apparatus according to claim 1, further comprising an operation unit configured to receive an input operation, wherein the operation unit includes a first operation unit configured to switch the first display state to the second display state in which the orientation of the subject is rotated clockwise so as to match the orientation of the subject in the X-ray image generated by the image generation unit, and a second operation unit configured to switch the first display state to the second display state in which the orientation of the subject is rotated counterclockwise so as to match the orientation of the subject in the X-ray image generated by the image generation unit.
